# EUROPEAN PATENT APPLICATION

(11) **EP 1 099 400 A2**
(43) Date of publication of application: **16.05.2001**
(21) Application number: 00124141.3
(22) Date of filing: 07.11.2000
(51) Int. Cl.: A47K 5/12

(54) **Improvements to an automatic device for disinfecting hands**

(30) Priority: 11.11.1999 IT MO990252
(71) Applicant: Gamar S.R.L., 42015 Correggio, (Reggio Emilia) (IT)
(72) Inventor: Mora, Giuliano, 42015 Correggio, (Reggio Emilia) (IT)
(74) Representative: Cerreta, Caterina

(57) **Abstract**

The object of the present invention falls into the field of automatic devices for disinfecting hands. A solenoid valve (7) is positioned on the route of a small tube (6) connecting a compressor (4) to at least one nozzle (3). The said solenoid valve is connected electrically to a proximity sensor, synchronically to the compressor (4). A battery (8) provides the electric current required for the functioning of the compressor (4), the solenoid valve (7) and the proximity sensor.

## Description

The invention relates to improvements to an automatic device for disinfecting hands.

For some time now it has become common to be able to disinfect one's hands in a many different places, whether a working environment, or recreational areas.

Think, for example, of the food industry, the pharmaceutical industry and hospitals, to remain in the field of working environments. Or sports centres, where the presence of a large number of people makes it increasingly important to pay attention to hygiene requirements.

In some environments, for example the aforementioned hospitals, there are not only hygiene requirements but also sterilisation requirements.

Consequently, for some time now, many devices have been designed and manufactured to cater for these disinfecting requirements quickly and efficiently.

The commonly known devices, all of which generally have the form of a container with an external niche designed for the positioning of one or both hands, have some drawbacks, one of which being the fact that the disinfecting jet is not activated at exactly the same moment as the hands are placed in the aforementioned niche. Another drawback of the commonly known devices lies in the fact that the installation of the aforementioned devices depends on the presence of a centralised electricity supply mains.

The first of the said drawbacks is determined by the fact that, at the end of each treatment, the flow of the liquid disinfectant does not remain constant inside the tube connecting the compressor of the said liquid to the spray elements and so this gradually creates a dispensing delay at the beginning of the next treatment.

Apart from the inconvenience of this drawback, it also has a practical repercussion on the efficiency itself of the disinfecting treatment. In fact, in the said devices, for the majority of cases, a timing circuit is envisaged which interrupts the dispensing of the liquid after a certain amount of time has lapsed.

This interruption takes place independently of whether or not the user's hands are still under the sensor for the detection of the said hands and this is to prevent the liquid disinfectant being wasted. It is obvious that, in spite of the fact that the duration of each treatment can be set beforehand, the short space of time lost at the beginning of the treatment itself penalises the device.

As to the second drawback of the commonly-known devices, i.e. the dependence on the presence of an external electricity supply, it is obvious how much this can influence both the number of devices present in an area and also the convenience and accessibility of these devices.

It should also be noted that in certain places, where there is either great humidity or water jets are used freely, the presence of a system for connection to an external electricity supply mains could constitute a serious hazard: this may lead some users to refuse the installation of the sanitising devices if they are only fitted with this type of power supply system.

A further drawback originating from the fact that the commonly known devices depend exclusively on an external electricity supply mains is due to the possibility that the said supply mains may be subject to interruptions of various kinds with the result that the sanitising devices are rendered unusable.

A first aim of this invention is to fit the said sanitising device with a means designed to guarantee the immediate dispensing of the liquid disinfectant.

A further aim of the present invention is to make available an automatic device for disinfecting hands, fitted with an electric battery.

In particular, the improvements to an automatic device for disinfecting hands (of the kind in question in the present invention, which has an external niche for the positioning of one or both the hands, a proximity sensor, a storage tank for the liquid disinfectant, a compressor, at least one dispensing nozzle, and a small tube connecting the aforementioned compressor to the nozzle) are characterised by the fact that they include:
- a solenoid valve positioned in the small tube which takes the liquid disinfectant from the first tank to the dispensing nozzle;
- a battery which generates the electrical current;

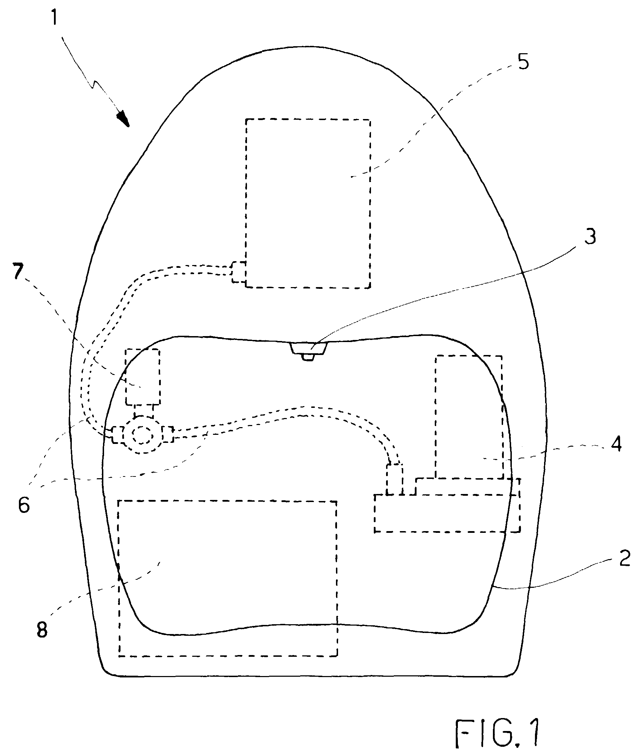

This and other characteristics will better emerge from the detailed description that follows of a preferred embodiment, provided in the form of a non-limiting example, with reference to the accompanying drawing, in which 1 illustrates a functional diagram of the automatic device.

With reference to the aforementioned figure, 1 denotes an automatic device for disinfecting hands in which there is a spray nozzle 3 positioned inside a niche 2 designed to contain, without contact, the said one or both hands.

This nozzle is connected to a compressor 4, which is connected, in its turn, to a storage tank 5 for the liquid disinfectant, by means of the small tube 6 running between the two.

Along the route of the said small tube there is a solenoid valve 7 positioned immediately between the compressor 4 and the nozzle 3. Inside the orifice 2 there is a proximity sensor, which is not shown. The said proximity sensor is connected electrically to the compressor 4 and the solenoid valve 7 in such a way that the electrical pulse determines, at the same time, both the start-up of the aforementioned compressor and the closure of the said solenoid valve.

A battery 8 constitutes the sole element to generate the electrical current necessary to supply the compressor 4, the solenoid valve 7 and the proximity sensor.

The functioning modes of the improvements which represent the object of the present invention will now be described with reference to numbers indicated in the figures.

Every time the proximity sensor detects the presence of at least one hand inside the niche 2, it activates, at the same moment, the functioning of the compressor 4 and the opening of the solenoid valve 7.

Only the first sanitising treatment will be subject to a delay in the liquid coming out of the nozzle 3, as it is necessary to wait for the aforementioned liquid disinfectant to fill the small tube 6.

When the second treatment begins, however, the said liquid will be dispensed immediately. In fact, when the first disinfecting treatment is finished, at the same time as the compressor 4, which pumps the said liquid along the small tube 6, is switched off, the solenoid valve 7 will also switch itself off to prevent the said small tube emptying, even partially. In this way, the flow of liquid disinfectant inside the small tube 6 will be available immediately at the nozzle 3 for the following treatment.

A first alternative embodiment envisages the insertion of the battery 8 into an electrical circuit, inside the device in question in the present invention, which can be connected to an external power supply.

In this case, the device object of the present invention is fitted with a double power supply option, which increases the installation flexibility of the said device.

A further embodiment envisages the use of a series of batteries, of the kind available in the shops, rather than the battery 8.

A first advantage of the improvements in question in the present invention lies in the fact that the delay at the beginning of the each hand disinfecting treatment after the initial treatment is prevented. Another advantage lies in the fact that the said automatic device can also function in places or positions which are not fitted with or are not close to external electricity supplies.

A further advantage of the present invention lies in the fact that the sanitsing device is not subject to any power failures caused by the external electricity supply mains.

## Claims

1. Improvements to an automatic device (1) for disinfecting hands, of the kind with an external niche (2) for the positioning of the one or both the said hands, with a proximity sensor, a storage tank (5) for the liquid disinfectant, a compressor (4), at least one nozzle (3), characterised by the fact that they include a solenoid valve (7) positioned on the small tube (6) which takes the liquid disinfectant from the compressor (4) to the dispensing nozzle (3) and a battery (8) which generates the electric current.

2. Improvements according to claim 1, characterised by the fact that the solenoid valve (7) is connected to the proximity sensor, as is the compressor (4); this said double electrical connection to the nozzle (3) being designed to close the said solenoid valve synchronically with the start-up of the compressor (4).

3. Improvements according to claim 1, characterised by the fact that the battery (8) can be inserted, with a parallel connection, into an electrical circuit inside the automatic device (1), fitted with suitable means of connection to the external power supply mains.

4. Improvements according to claim 1, characterised by the fact that instead of the battery (8), a series of smaller batteries could be used, of the kind available in the shops.
